# EUROPÄISCHE PATENTANMELDUNG

(11) **EP 1 980 283 A1**
(43) Veröffentlichungstag der Anmeldung: **15.10.2008**
(21) Anmeldenummer: 07007306.9
(22) Anmeldetag: 10.04.2007
(51) Int. Cl.: A61M 5/142

(54) **Medikamentenreservoir und Vorrichtung zur automatisierten Abgabe eines flüssigen Medikaments**

(71) Anmelder: F.HOFFMANN-LA ROCHE AG, 4070 Basel (CH); Roche Diagnostics GmbH, 68305 Mannheim (DE)
(72) Erfinder: Zihlmann, Rudolf, 3550 Langnau (CH); Oberli, Markus, 3422 Kirchberg (CH); Remde, Axel, 3432 Lützelflüh-Goldbach (CH); Scheurer, Simon, 3006 Bern (CH); Horisberger, Ronny, 3424 Oberösch (CH); Haueter, Ulrich, 3506 Grosshöchstetten (CH); Heutschi, Ivan, 3506 Grosshöchstetten (CH)
(74) Vertreter: Schalch, Rainer

(57) **Zusammenfassung**

Die Erfindung betrifft ein Medikamentenreservoir (1) mit einem Behältnis (2) zur Aufnahme eines flüssigen Medikaments, welches an seiner Stirnwand (4) einen Medikamentenaustritt (5) aufweist. Der Behältnisinnenraum (3) ist durch einen längsverschiebbaren Verdrängerkolben (6) fluiddicht nach aussen abgeschottet, wobei der Verdrängerkolben (6) innerhalb des Behältnisses (2) längsverschiebbar ist, so dass es bei mit einem flüssigen Medikament gefülltem Behältnisinnenraum (3) möglich ist, durch Verschieben des Verdrängerkolbens (6) flüssiges Medikament aus dem Behältnisinnenraum (3) in den Medikamentenaustritt (5) zu verdrängen. Dabei ist das Behältnis (2) derartig ausgebildet, dass der Verdrängerkolben (6) in der Verschiebeendposition, welche er am Ende der Medikamentenförderung bei maximal entleertem Medikamentenreservoir (1) einnimmt, an die Stirnwand (4) des jeweiligen Behältnisses (2), von welcher der gemeinsame Medikamentenaustritt (5) nach aussen führt, angrenzt. Die Austrittsrichtung aus dem Medikamentenaustritt (5) verläuft senkrecht zur Verschieberichtung des Verdrängerkolbens (6).

Durch das erfindungsgemässe Medikamentenreservoir (1) wird die Bereitstellung von Vorrichtungen zum automatisierten Abgeben flüssiger Medikamente ermöglicht, welche gegenüber den heute bekannten Vorrichtungen mit etablierter Architektur eine deutlich verkürzte Baulänge aufweisen.

## Beschreibung

Die vorliegende Erfindung betrifft ein Medikamentenreservoir, eine Vorrichtung zum automatisierten Abgeben eines flüssigen Medikaments mit einem solchen Medikamentenreservoir sowie ein Set umfassend ein solches Medikamentenreservoir und eine Aufziehhilfe gemäss den Oberbegriffen der unabhängigen Patentansprüche.

Vorrichtungen zum automatisierten Abgeben von flüssigen Medikamenten kommen bevorzugt bei Patienten zur Anwendung, welche einen kontinuierlichen und über den Tag gesehen variierenden Bedarf an einem subkutan verabreichbaren Medikament haben. Konkrete Anwendungsfälle sind z.B. bestimmte Schmerztherapien und die Behandlung von Diabetes, bei denen am Körper tragbare computergesteuerte Medikamentenpumpen, z.B. Insulinpumpen, zum Einsatz kommen, welche oftmals eine für mehrere Tage ausreichende Medikamentenmenge in einem Medikamentenreservoir beinhalten und das flüssige Medikament gemäss einem vorprogrammierten Tagesprofil vom Medikamentenreservoir über eine Infusionskanüle in den Körper des Patienten fördern. Dabei besteht zunehmend das Bedürfnis, die Pumpe unsichtbar zu tragen, wozu diese möglichst kleine Abmessungen und insbesondere eine möglichst geringe Baulänge aufweisen sollte, um durch die Bekleidung nicht wahrgenommen zu werden und einen möglichst guten Tragekomfort zu bieten.

Die heute bekannten, am Körper tragbaren Medikamentenpumpen weisen als Medikamentenreservoir eine zumeist zylindrische Ampulle mit einem darin angeordneten Verdrängerkolben auf, welcher mittels einer Kolbenstange oder Gewindespindel in die Ampulle geschoben werden kann, zur Förderung des Medikaments. Die Konnektierung einer abführenden Medikamentenförderleitung oder eines Konnektierungsadapters einer solchen Medikamentenförderleitung erfolgt dabei in Längsrichtung der Ampulle bzw. entlang der Verschiebeachse des Verdrängerkolbens. Diese heute bekannten Ausführungsformen weisen den Nachteil auf, dass sie eine unerwünscht grosse Baulänge aufweisen, was der Bereitstellung kompakter Medikamentenpumpen abträglich ist.

Es stellt sich die Aufgabe, ein Medikamentenreservoir und eine Vorrichtung zum automatisierten Abgeben eines flüssigen Medikaments zur Verfügung zu stellen, welche die Nachteile des Standes der Technik nicht aufweisen oder zumindest teilweise vermeiden.

Diese Aufgabe wird durch das Medikamentenreservoir und die Vorrichtung gemäss den unabhängigen Patentansprüchen gelöst.

Ein erster Aspekt der Erfindung betrifft ein Medikamentenreservoir mit einem oder mehreren Behältnissen, welches oder welche zur Aufnahme eines flüssigen Medikaments vorgesehen ist oder sind bzw. bereits ein flüssiges Medikament enthält oder enthalten. Als flüssige Medikamente kommen beispielsweise flüssige Schmerzmittel oder Insulin in Frage. Jedes Behältnis weist einen Behältnisinnenraum mit einer Stirnwand auf, von welcher ein Medikamentenaustritt direkt oder über ein oder mehrere weitere Behältnisse nach aussen führt. Der Medikamentenaustritt kann z.B. durch ein Septum verschlossen sein, welches zur Ermöglichung einer Abgabe von flüssigem Medikament mit einer Kanüle durchstochen werden muss, um einen Durchtritt nach aussen zu erhalten. Der Behältnisinnenraum ist durch einen längsverschiebbaren Verdrängerkolben fluiddicht nach aussen abgeschottet. Der Verdrängerkolben grenzt in radialer Richtung über seinen gesamten Aussenumfang fluiddicht an diesen umschliessende Begrenzungswandungen des Behältnisses an, wobei das Behältnis derartig ausgestaltet ist, dass der Verdrängerkolben unter Beibehaltung seiner fluiddichten Angrenzung an die Begrenzungswandungen innerhalb des Behältnisses längsverschiebbar ist. Hierdurch ist es möglich, das Volumen des Behältnisinnenraums durch Verschieben des Verdrängerkolbens innerhalb des Behältnisses zu verkleinern, unter Verdrängung von Fluid aus dem Behältnisinnenraum. Entsprechend ist es bei mit einem flüssigen Medikament gefülltem Behältnisinnenraum und nach aussen geöffnetem Medikamentenaustritt möglich, durch Verschieben des Verdrängerkolbens flüssiges Medikament aus dem Behältnisinnenraum zu Verdrängen und in den Medikamentenaustritt zu fördern. Weiter ist jedes Behältnis derartig ausgebildet, dass der Verdrängerkolben in der Verschiebeendposition, welche er am Ende der Medikamtenförderung bei maximal entleertem Medikamentenreservoir einnimmt, an die Stirnwand des Behältnisses, von welcher der Medikamentenaustritt nach aussen führt, angrenzt, wobei er bevorzugterweise grossflächig an dieser anliegt. Dabei ist die bestimmungsgemässe Austrittsrichtung aus dem oder den Medikamentenaustritten bzw. bei mehreren Behältnissen mit einem gemeinsamen Medikamentenaustritt aus diesem gemeinsamen Medikamentenaustritt quer, d.h. unter einem Winkel, z.B. unter einem Winkel von 100°, bevorzugterweise senkrecht zur Verschieberichtung des dem jeweiligen Behältnis zugeordneten Verdrängerkolbens oder bei mehreren Behältnissen mit einem gemeinsamen Medikamentenaustritt quer bzw. senkrecht zur Verschieberichtung zumindest eines der Verdrängerkolben.

Durch das erfindungsgemässe Medikamentenreservoir wird die Bereitstellung von Vorrichtungen zum automatisierten Abgeben flüssiger Medikamente ermöglicht, welche gegenüber den heute bekannten Vorrichtungen etablierter Architektur eine deutlich verkürzte Baulänge aufweisen.

In einer bevorzugten Ausführungsform des Medikamentenreservoirs ist die für die Verdrängung wirksame Fläche des jeweiligen Verdrängerkolbens, also seine in Verschieberichtung projizierte Fläche, die gesamte Fläche, welche von seinem Aussenumfang bzw. von den seinen Aussenumfang umschliessenden Begrenzungswandungen umschlossen wird. Solche Medikamentenreservoirs sind kostengünstig in der Herstellung, weisen ein maximales Medikamentenvolumen bei gegebenem Verschiebeweg des Verdrängerkolbens auf und sind zudem aus hygienetechnischer Sicht zu bevorzugen, da lediglich eine umfangsmässige Abdichtung zwischen dem Verdrängerkolben und den Begrenzungswandungen des Behältnisses erforderlich ist, welche auf einfache und erprobte Weise zu bewerkstelligen ist.

In noch einer bevorzugten Ausführungsform des Medikamentenreservoirs kann eine Medikamentenförderleitung direkt an den Medikamentenaustritt des Medikamentenreservoirs angekoppelt werden oder ist an diesen fest oder abnehmbar angeschlossen. Hierdurch kann das Medikamentenreservoir zusammen mit dem Infusionsset als kostengünstiges und hygienetechnisch optimales Einwegteil bereitgestellt werden.

In noch einer weiteren bevorzugten Ausführungsform des Medikamentenreservoirs ist bei Ausführungsformen mit einem einzigen Behältnis der Verdrängerkolben des Medikamentenreservoirs derartig ausgestaltet, dass die kleinste Erstreckung seiner für die Verdrängung wirksamen Fläche kleiner ist als der Durchmesser eines Zylinders, welcher bei einer Höhe entsprechend dem für die Medikamentenförderung vorgesehenen Verschiebeweges des Verdrängerkolbens ein Volumen entsprechend dem vorgesehenen Fördervolumen des Medikamentenreservoirs aufweist. Bei Ausführungsformen mit mehreren Behältnissen sind die Verdrängerkolben des Medikamentenreservoirs derartig ausgestaltet, dass die grösste von den jeweils kleinsten Erstreckungen der für die Verdrängung wirksamen Flächen der Verdrängerkolben kleiner ist als der Durchmesser eines Zylinders, welcher bei einer Höhe entsprechend dem längsten der für die Medikamentenförderung vorgesehenen Verschiebewege der Verdrängerkolben ein Volumen entsprechend dem vorgesehenen Fördervolumen des Medikamentenreservoirs aufweist. Hierdurch wird die Bereitstellung von Vorrichtungen zum automatisierten Abgeben flüssiger Medikamente ermöglicht, welche extrem flach sind und gleichzeitig hohen hygienetechnischen Anforderungen gerecht werden.

In noch einer weiteren bevorzugten Ausführungsform des Medikamentenreservoirs weist dieses genau ein Behältnis zur Aufnahme eines flüssigen Medikaments bzw. enthaltend ein flüssiges Medikament auf. Solche Medikamentenreservoirs lassen sich kostengünstig bereitstellen und sind aufgrund ihres einfachen Aufbaus besonders funktionssicher.

In einer anderen bevorzugten Ausführungsform des Medikamentenreservoirs umfasst dieses mehrere, insbesondere identische Behältnisse, und zwar bevorzugterweise genau zwei oder genau drei bevorzugterweise identische Behältnisse. Durch gezielte Auswahl einer bestimmten Konfiguration kann bei gegebenem Medikamentenvolumen in weiten Bereichen Einfluss auf die Abmessungen des Medikamentenreservoirs und damit auf die Abmessungen einer für die Aufnahme des Reservoirs vorgesehenen Medikamentenpumpe genommen werden. So lassen sich z.B. extrem flache Medikamentenreservoirs zur Verfügung stellen, welche zudem extrem kompakt sein können, z.B. im Fall von parallel nebeneinander angeordneten identischen Behältnissen mit quadratischem Querschnitt.

In noch einer weiteren bevorzugten Ausführungsform des Medikamentenreservoirs weist zumindest ein Behältnis, bevorzugterweise alle Behältnisse des Medikamentenreservoirs jeweils einen Verdrängerkolben auf, dessen für die Verdrängung wirksame Fläche kreisrund ist. Diese Bauweise ist bevorzugt, da sich hierdurch höchste Anforderungen an Dichtheit und Hygiene erfüllen lassen und zudem eine einfache und kostengünstige Herstellung möglich ist.

In noch einer weiteren bevorzugten Ausführungsform des Medikamentenreservoirs weist zumindest ein Behältnis, bevorzugterweise alle Behältnisse des Medikamentenreservoirs jeweils einen Verdrängerkolben auf, dessen für die Verdrängung wirksame Fläche eine nicht-kreisrunde Fläche, und zwar bevorzugterweise eine ovale, knochenförmige, langlochförmige, rechteckige, dreieckige oder hexagonale Fläche ist, wobei letztgenannte bevorzugterweise eine flache bzw. gestreckte hexagonale Form aufweist. Hierdurch ergibt sich der Vorteil, dass durch gezielte Auswahl einer bestimmten Bauform und Konfiguration bei gegebenem Medikamentenvolumen in weiten Bereichen Einfluss auf die Dicke des Medikamentenreservoirs und damit Einfluss auf die Abmessungen einer für die Aufnahme des Reservoirs vorgesehenen Medikamentenpumpe genommen werden kann. Dabei ist es zur Stabilisierung des Behältnisses auch vorgesehen, die Behältniswandungen, welche an die längeren Seiten der für die Verdrängung wirksamen Fläche des Verdrängerkolbens angrenzen, auf ihrer Aussenseite mit quer zur Verschieberichtung des Verdrängerkolbens verlaufenden Stützrippen oder mit einer erhöhten Wandstärke zu versehen, so dass diese einer Durchbiegeung nach Aussen unter Innendruck ein erhöhtes Biegewiderstandsmoment entgegensetzen. Dies ist insbesondere bei Verdrängerkolben mit einer knochenförmigen für die Verdrängung wirksamen Fläche von Vorteil, weil diese Massnahme hier anwendbar ist, ohne die Gesamtdicke des Medikamentenreservoirs zu erhöhen.

In noch einer weiteren bevorzugten Ausführungsform des Medikamentenreservoirs umfasst dieses mindestens zwei Behältnisse, wobei die Verdrängerkolben der Behältnisse derartig mechanisch miteinander gekoppelt sind, dass deren Verschiebung zwangsläufig gleichzeitig erfolgen muss. Hierdurch ergibt sich der Vorteil, dass eine Förderung aus sämtlichen Behältnissen mittels einer an einem gemeinsamen Krafteinleitungspunkt angreifenden Antriebseinheit möglich ist, wodurch bei der Bereitstellung einer Medikamentenpumpe mit einem derartigen Medikamentenreservoir Kosten und Bauraum eingespart werden können.

Weiter ist es bei erfindungsgemässen Medikamentenreservoirs, welche mehrere Behältnisse umfassen, bevorzugt, dass die Behältnisse mit ihren Längsachsen, also mit den Verschiebeachsen der Verdrängerkolben, parallel zueinander angeordnet sind, insbesondere derart, dass die Längsachsen sämtlicher Behältnisse in einer Ebene angeordnet sind. Hierdurch lassen sich Bauformen mit spezifischen Abmessungen realisieren.

Weiter ist es bei erfindungsgemässen Medikamentenreservoirs, welche mehrere Behältnisse umfassen, bevorzugt, dass die Behältnisse fluidisch miteinander verbunden sind, d.h. ihre Behältnisinnenräume miteinander kommunizieren. Hierdurch verhalten sich die Behältnisse zusammen wie ein einziges Behältnis.

Weiter ist es bei erfindungsgemässen Medikamentenreservoirs, welche mehrere Behältnisse umfassen, bevorzugt, wenn die Behältnisse einstückig miteinander ausgebildet sind, wobei es bevorzugt ist, wenn diese einen gemeinsamen, einstückig mit diesen ausgebildeten Medikamentenaustritt aufweisen. Derartige Medikamentenreservoirs sind robust, betriebssicher und kostengünstig in der Herstellung.

Alternativ zu der zuvor erwähnten Ausführungsform ist es bei erfindungsgemässen Medikamentenreservoirs, welche mehrere Behältnisse umfassen, bevorzugt, wenn die Behältnisse als separate Bauteile ausgebildet sind, wobei es bevorzugt ist, wenn diese über ein weiteres Bauteil fluidisch miteinander gekoppelt sind, bevorzugterweise derart, dass deren Medikamentenaustritte miteinander kommunizieren. Hierdurch wird es möglich, aus einer beschränkten Anzahl verschiedener Behältnisse und Kopplungsadapter eine Vielzahl verschiedenartiger erfindungsgemässer Medikamentenreservoirs zu konfektionieren.

In einer weiteren bevorzugten Ausführungsform ist es bei Medikamentenreservoirs, welche mehrere Behältnisse umfassen, bevorzugt, wenn zwischen zwei benachbarten Behältnissen ein Zwischenraum vorhanden ist. Auf diese Weise besteht für die Ausgestaltung von Medikamentenpumpen mit solchen Medikamentenreservoirs die Möglichkeit, Bauelemente insbesondere der Antriebseinheit für die Betätigung der Verdrängerkolben, wie z.B. ein Motor mit Antriebsspindel und/oder Getriebe, zwischen den Behältnissen zu platzieren und auf diese Weise Baulänge einzusparen. Zudem wird hierdurch eine symmetrische Anordnung der Behältnisse um den Krafteinleitungspunkt der Antriebseinheit herum ermöglicht, wodurch ein verkantungsfreier Betrieb der aus Antriebseinheit und Verdrängerkolben gebildeten Bewegungsanordnung begünstigt wird.

In noch einer weiteren bevorzugten Ausführungsform des Medikamentenreservoirs ist jeder Verdrängerkolben, vorteilhafterweise durch einstückige Ausbildung, starr mit einer zugeordneten Kolbenstange verbunden.

Dabei ist es bevorzugt, dass die Kolbenstange im Medikamentenreservoir bzw. im jeweiligen den zugeordneten Verdrängerkolben aufnehmenden Behältnis von den Behältniswandungen radial geführt wird, so dass die Baulänge des eigentlichen Verdrängerkolbens so weit reduziert werden kann, dass dieser nur noch Abdichtfunktionen wahrnimmt, nicht jedoch durch seine Baulänge an einem Abkippen gehindert wird. Letztgenannte Funktion übernimmt in diesem Fall die geführte Kolbenstange. Hierdurch lässt sich wiederum Baulänge einsparen.

Auch ist es bei den beiden zuvor erwähnten Ausführungsformen bevorzugt, wenn die Kolbenstange aus einem härteren Material gebildet ist als der Verdrängerkolben, um deren unterschiedlichen Funktionen Rechnung zu tragen. Dabei ist es von Vorteil ist, wenn die Kolbenstange und der Verdrängerkolben durch Mehrkomponentenspritzguss einstückig miteinander ausgebildet sind. Auf diese Weise können diese Komponenten in Grossserie in hochwertiger Qualität zu geringen Stückkosten bereitgestellt werden.

Des Weiteren ist es bei erfindungsgemässen Medikamentenreservoirs mit mindestens zwei Behältnissen bevorzugt, dass die Kolbenstangen der Verdrängerkolben durch bevorzugterweise einstückige Ausbildung derartig miteinander verbunden sind, dass ein Verschieben der Verdrängerkolben zwangsläufig gleichzeitig um den gleichen Betrag erfolgen muss. Auf diese Weise kann ein kostengünstiges und funktionssicheres Medikamentenreservoir zur Verfügung gestellt werden, welches seitens der mit diesem zu bildenden Medikamentenpumpe nur ein Minimum an pumpeninternen Bauteilen für den Antrieb der Verdrängerkolben erfordert.

In noch einer weiteren bevorzugten Ausführungsform des Medikamentenreservoir umfasst dieses einen oder mehrere Anschläge, welche den Verschiebeweg des oder der Verdrängerkolben in einer Richtung entgegen der zur Medikamentenförderung vorgesehenen Verschieberichtung formschlüssig begrenzen. Hierdurch wird verhindert, dass der Verdrängerkolben beim Zurückziehen innerhalb des Behältnisses, z.B. bei einem Befüllen des Medikamentenreservoirs, aus dem Behältnis herausgezogen wird, was aus hygienetechnischer Sicht problematisch wäre.

In noch einer weiteren bevorzugten Ausführungsform ist das Medikamentenreservoir mit Insulin befüllt, bevorzugterweise mit einer Insulinmenge entsprechend mindestens 150 IU (Insulin-Einheiten), noch bevorzugter entsprechend mindestens 300 IU (Insulin-Einheiten). Dies entspricht bei dem heute für solche Pumpen gebräuchlichen Insulin U100 einer Füllmenge von mindestens 1,5 ml bzw. 3 ml Insulin. Solche Medikamentenreservoirs stellen, insbesondere in Form von Einwegabpackungen, bevorzugte Handelswaren dar.

Ein zweiter Aspekt der Erfindung betrifft eine Vorrichtung zum automatisierten Abgeben eines flüssigen Medikaments, auch als Medikamentenpumpe bzw. im Fall der Abgabe von Insulin als Insulinpumpe bezeichnet. Die erfindungsgemässe Vorrichtung umfasst ein Medikamentenreservoir gemäss dem ersten Aspekt der Erfindung, zur Bereitstellung eines flüssigen Medikaments sowie eine mit einer bevorzugterweise elektronischen Steuerung versehene Antriebseinheit, mittels welcher der Verdrängerkolben, oder, bei einem Medikamentenreservoir mit mehreren Verdrängerkolben, die Verdrängerkolben des Medikamentenreservoirs im jeweiligen sie aufnehmenden Behältnis verschoben werden können, zur Bewirkung einer Förderung von flüssigem Medikament aus dem Behältnis.

Solche erfindungsgemässen Medikamentenpumpen können sehr kompakt ausgebildet werden und erfüllen gleichzeitig hohe hygienetechnische Anforderungen.

In einer bevorzugten Ausführungsform der Vorrichtung verläuft die Anschlussrichtung zum Anschliessen einer Medikamentenförderleitung an die Vorrichtung quer, bevorzugterweise senkrecht zur Verschieberichtung des Verdrängerkolbens oder bei mehreren Verdrängerkolben senkrecht zur Verschieberichtung zumindest eines der Verdrängerkolben. Dabei ist es bevorzugt, wenn die Vorrichtung derartig ausgestaltet ist, dass die Medikamentenförderleitung direkt an den Medikamentenaustritt des Medikamentenreservoirs anschliessbar bzw. angeschlossen ist. Hierdurch ergibt sich der Vorteil, dass zur Konnektierung der Medikamentenförderleitung nur ein Minimum an Bauteilen erforderlich ist, wodurch sich Kosten einsparen lassen.

In einer weiteren bevorzugten Ausführungsform weist die erfindungsgemässe Vorrichtung ein Medikamentenreservoir mit mindestens zwei Behältnissen auf, wobei die Verdrängerkolben der Behältnisse derartig mechanisch miteinander gekoppelt sind, dass deren Verschiebung zwangsläufig gleichzeitig erfolgen muss, bevorzugterweise um einen identischen Betrag. Durch diese Bauweise können derartige Vorrichtungen kostengünstig und in robuster Ausführung zur Verfügung gestellt werden.

In noch einer weiteren bevorzugten Ausführungsform weist die Vorrichtung ein Medikamentenreservoir mit mindestens zwei Behältnissen auf, wobei zwischen zwei benachbarten Behältnissen des Medikamentenreservoirs ein Zwischenraum vorhanden ist, in welchem zumindest ein Teil der Antriebseinheit angeordnet ist, und zwar bevorzugterweise ein Antriebsmotor, eine Antriebsspindel und/oder ein Getriebe der Antriebseinheit. Auf diese Weise kann Baulänge eingespart werden und es wird möglich, die Behältnisse symmetrisch um den Krafteinleitungspunkt der Antriebseinheit herum anzuordnen, wodurch ein verkantungsfreier Betrieb begünstigt wird.

In noch einer bevorzugten Ausführungsform der Vorrichtung ist die Vorrichtung eine am Körper eines Patienten tragbare Insulinpumpe. Bei solchen Ausgestaltungen der Vorrichtung treten die Vorteile der Erfindung besonders deutlich zu Tage.

Ein dritter Aspekt der Erfindung betrifft ein Set umfassend ein Medikamentenreservoir gemäss dem ersten Aspekt der Erfindung und eine Aufziehhilfe, welche an das Medikamentenreservoir angekoppelt werden kann, zum Zurückziehen des oder der Verdrängerkolben zwecks Befüllung des Medikamentenreservoirs. Hierdurch wird die Befüllung des Medikamentenreservoirs mit flüssigem Medikament aus einer Grossabpackung deutlich erleichtert.

Weitere bevorzugte Ausführungen der Erfindung ergeben sich aus den abhängigen Ansprüchen und aus der nun folgenden Beschreibung anhand der Figuren. Dabei zeigen:
Fig. 1 eine perspektivische Draufsicht auf ein erstes erfindungsgemässes Medikamentenreservoir;
Fig. 2 eine Längsschnitt durch das Medikamentenreservoir aus Fig. 1 mit dem Verdrängerkolben in der hinteren Verschiebeendposition;
Fig. 3 eine Darstellung wie Fig. 2 mit dem Verdrängerkolben in einer vorderen Verschiebeposition;
Fig. 4 eine perspektivische Draufsicht auf den Verdrängerkolben des Medikamentenreservoirs aus Fig. 1;
Fig. 5 einen Schnitt entlang der Linie A-A in Fig. 2;
die Figuren 5a und 6 bis 8 Darstellungen wie Fig. 5 durch bevorzugte Ausführungsvarianten des erfindungsgemässen Medikamentenreservoirs aus Fig. 1;
Fig. 9 eine Darstellung wie Fig. 4 einer bevorzugten Ausführungsvariante des Verdrängerkolbens des Medikamentenreservoirs aus Fig. 1;
Fig. 10 eine perspektivische Draufsicht auf eine Aufziehhilfe für ein erfindungsgemässes Medikamentenreservoir mit dem Verdrängerkolben aus Fig. 9;
Fig. 11 eine perspektivische Draufsicht auf die Aufziehhilfe aus Fig. 10 und den Verdrängerkolben aus Fig. 9 im zusammengekoppelten Zustand;
Fig. 12 eine perspektivische Draufsicht auf ein erfindungsgemässes Medikamentenreservoir mit dem Verdrängerkolben aus Fig. 9 nach den Vorschieben des Verdrängerkolbens in den vorderen Verschiebeendpunkt und dem anschliessenden Ankoppeln der Aufziehhilfe aus Fig. 10;
Fig. 13 einen Längsschnitt durch die Anordnung aus Fig. 12;
Fig. 14 eine perspektivische Draufsicht auf das Medikamentenreservoir aus Fig. 12 nach dem Zurückziehen des Verdrängerkolbens mit der Aufziehhilfe in den hinteren Verschiebeendpunkt;
Fig. 15 einen Längsschnitt durch die Anordnung aus Fig. 14;
Fig. 16 eine perspektivische Draufsicht auf ein zweites erfindungsgemässes Medikamentenreservoir;
Fig. 17 eine Längsschnitt durch das Medikamentenreservoir aus Fig. 16 mit den Verdrängerkolben angeordnet in einer vorderen Verschiebeposition;
Fig. 18 eine Darstellung wie Fig. 17 mit den Verdrängerkolben angeordnet in einer hinteren Verschiebeposition;
Fig. 19 eine perspektivische Draufsicht auf die Verdrängerkolben des Medikamentenreservoirs aus Fig. 16;
Fig. 20 einen Schnitt entlang der Linie B-B in Fig. 18;
die Figuren 21 bis 23 Darstellungen wie Fig. 20 durch bevorzugte Ausführungsvarianten des erfindungsgemässen Medikamentenreservoirs aus Fig. 16; und
Fig. 24 einen Längsschnitt durch ein drittes erfindungsgemässes Medikamentenreservoir.

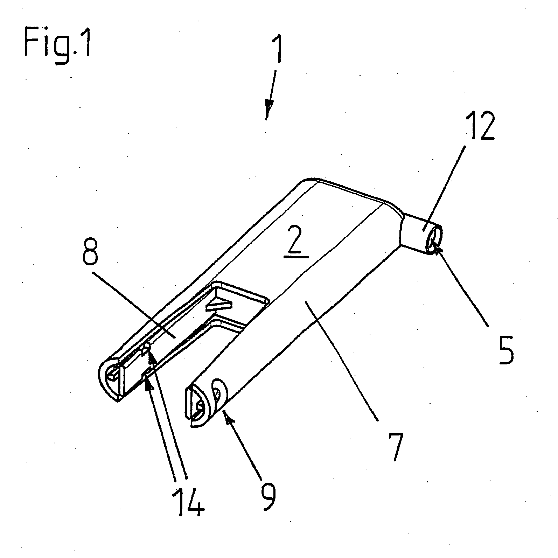

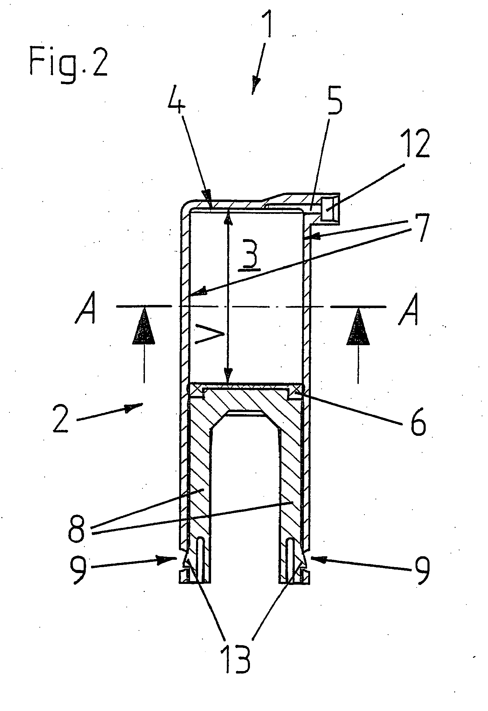

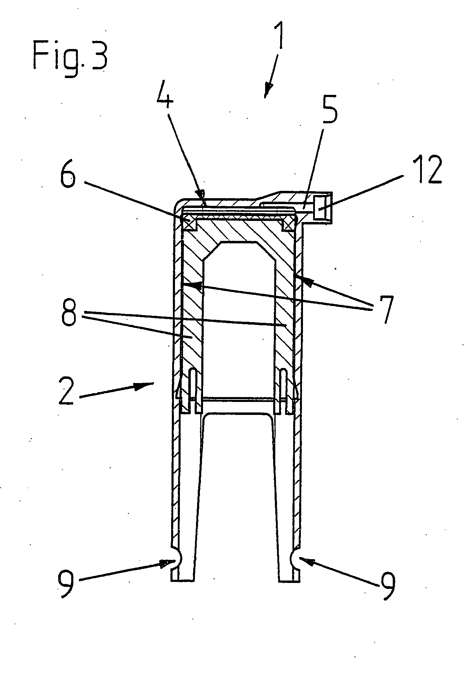

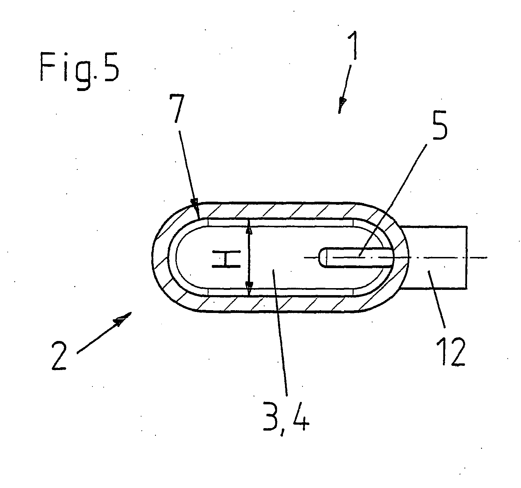

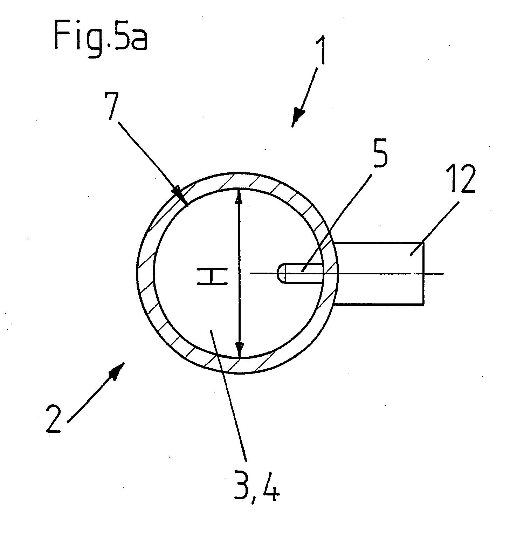

Eine erste bevorzugte Ausführungsform eines erfindungsgemässen Medikamentenreservoirs in Form einer Insulinampulle für eine Insulinpumpe ist in den Figuren 1 bis 5 dargestellt. Wie zu erkennen ist, umfasst das Medikamentenreservoir 1 ein Behältnis 2, innerhalb dessen Begrenzungswandungen 7 ein Verdrängerkolben 6 längsverschiebbar angeordnet ist, zum Verdrängen von Flüssigkeit aus dem Behältnisinnenraum 3. Das Behältnis 2 weist einen ovalen Querschnitt und der Verdrängerkolben 6 eine entsprechend ovale, für die Verdrängung wirksame Fläche auf, wobei die kleinste Erstreckung der für die Verdrängung wirksamen Kolbenfläche dem Abstand H der beiden parallelen Begrenzungswandungen entspricht (siehe Fig. 5). Der Behältnisinnenraum 3 weist eine Stirnwand 4 auf, an welcher ein Medikamentenaustritt 5 mit einer bestimmungsgemässen Austrittsrichtung, welche senkrecht zur Verschieberichtung des Verdrängerkolbens 6 verläuft, nach aussen führt. Der Medikamentenaustritt 5 mündet in einen Anschlussstutzen 12, an welchen eine Medikamentenförderleitung (nicht gezeigt) direkt anschliessbar ist. Der Verdrängerkolben 6, welcher zur Abdichtung gegenüber den Begrenzungswandungen 7 des Behältnisses 2 aus einem weichen Material, im vorliegenden Fall aus einem thermoplastischen Elastomer, gebildet ist, wird von einem zwei Kolbenstangen 8 bildenden Tragkörper aus einem verhältnismässig harten Material, im vorliegenden Fall PP (Polypropylen), getragen. Verdrängerkolben 6 und Tragkörper sind im vorliegenden Fall einstückig durch Kehrkomponentenspritzguss hergestellt. Es ist jedoch ebenso denkbar, einen Tragkörper mit Kolbenteil aus dem harten Material herzustellen und am Umfang des Kolbenteils eine Dichtung, z.B. einen O-Ring, zur radialen Abdichtung gegenüber den Begrenzungswandungen 7 anzuordnen. Wie aus einer Zusammenschau der Figuren 2 und 3 erkennbar wird, welche den Kolben 6 einmal in seiner bestimmungsgemäss maximal zurückgezogenen Position zeigt (Fig. 2), in welcher der Abstand V zwischen Kolbenvorderseite und der Stirnwand 4 dem für die Medikamentenförderung vorgesehenen Verschiebeweg V entspricht und der Behältnisinnenraum 3 somit das vorgesehene Fördervolumen des Medikamentenreservoirs 1 aufweist, und einmal in einer fast völlig eingeschobenen Position (Fig. 3), in welcher das Volumen des Behältnisinnenraumes 3 fast Null ist, kann der Verdrängerkolben 6 mittels des Tragkörpers bzw. der von diesem gebildeten Kolbenstangen 8 in das Behältnis 2 eingeschoben werden, zur Verdrängung eines Medikaments aus dessen Innenraum 3 und durch den Medikamentenaustritt 5 nach aussen. Dabei wird der Tragkörper innerhalb des Behältnisses 2 mit den Kolbenstangen 8 an den Begrenzungswandungen 7 geführt, so dass ein Verkanten bzw. Abkippen des Verdrängerkolbens 6 sicher verhindert wird. Wie zu erkennen ist, weisen die beiden Kolbenstangen 8 im Bereich ihrer freien Enden jeweils eine Federnase 13 auf, welche in der in Fig. 2 gezeigten vollständig zurückgezogenen Position des Verdrängerkolbens 6 in zugeordnete Ausnehmungen 9 in den Begrenzungswandungen 7 einrasten und so den Verschiebeweg des Verdrängerkolbens in einer Richtung entgegen der zur Medikamentenförderung vorgesehen Richtung, in welcher dieser beispielsweise zum Befüllen des Medikamentenreservoirs bewegt wird, formschlüssig begrenzen. Wie weiter zu erkennen ist, weisen die beiden Kolbenstangen 8 im Bereich ihrer freien Enden zudem jeweils zwei Ausnehmungen 14 auf, in welche Rastnasen einer Aufziehhilfe (nicht gezeigt) eingerastet werden können, zum Ankoppeln der Aufziehhilfe an das Medikamentenreservoir 1.

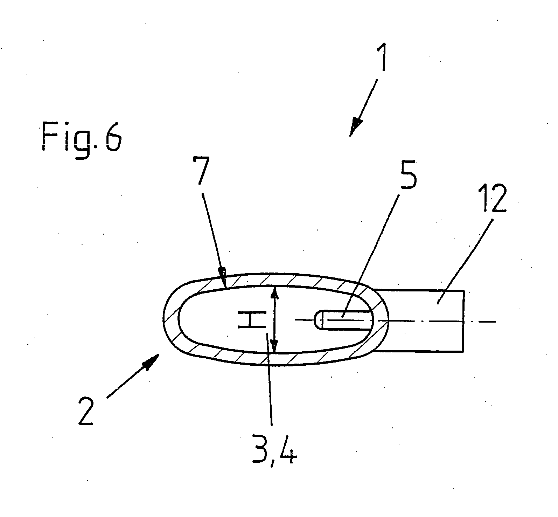

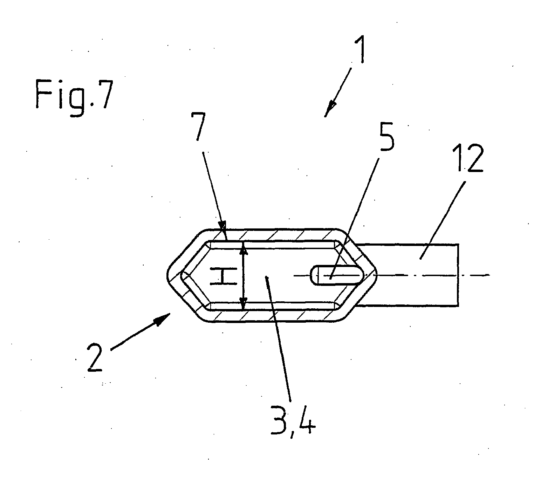

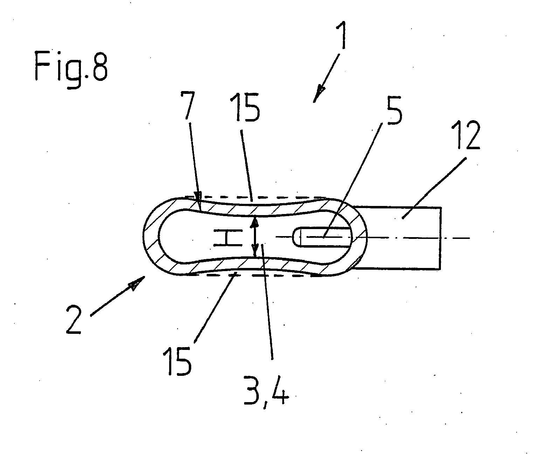

Die Figuren 5a und 6 bis 8 zeigen Darstellungen wie Fig. 5 durch bevorzugte Ausführungsvarianten des erfindungsgemässen Medikamentenreservoirs aus Fig. 1, welche andere Querschnittsformen aufweisen. Wie zu erkennen ist, weist die Ausführungsform gemäss Fig. 5a einen kreisrunden Querschnitt auf. Die Ausführungsformen gemäss den Figuren 6 und 8 weisen ovale Querschnittsformen auf, jedoch ohne parallele Begrenzungswandungen 7. Bei der in Fig. 6 gezeigten Ausführungsform sind die Begrenzungswandungen ausschliesslich konkav zum Innenraum 3 hin ausgebildet, was vorteilhaft hinsichtlich der Formstabilität bei einem Druckaufbau im Innenraum 3 ist. Bei der in Fig. 8 gezeigten Ausführungsform sind die Begrenzungswandungen zum Innenraum 3 hin abwechselnd einmal konvex und einmal konkav ausgebildet, wobei es hier bevorzugt ist, auf der Aussenseite der konvexen Begrenzungswandungen Stützrippen 15 anzubringen (gestrichelt dargestellt), welche einem Nachgeben der Wandungen nach Aussen bei Druckaufbau im Innenraum 3 des Behältnisses 2 entgegenwirken. Die kleinsten Erstreckungen H der für die Verdrängung wirksamen Flächen der Verdrängerkolben 6 erstrecken sich bei diesen beiden Ausführungsformen jeweils entlang der kürzesten Symmetrieachse der jeweiligen Querschnittsform. Die Ausführungsform gemäss Fig. 7 weist eine gestreckt sechseckige Querschnittsform auf, wobei hier die kleinste Erstreckung H der für die Verdrängung wirksamen Fläche des Verdrängerkolbens der Abstand zwischen den längsten parallelen Begrenzungswandungen 7 ist.

Die übrige Ausgestaltung dieser Medikamentenreservoirs ist, abgesehen von der anderen Querschnittsform des Behältnisses 2, identisch zu der in den Figuren 1 bis 5 gezeigten.

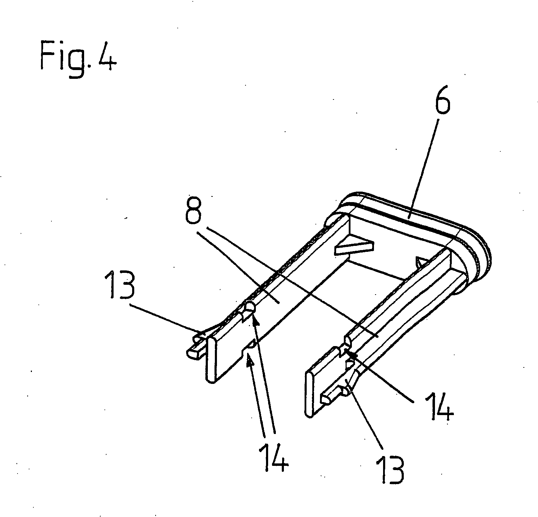

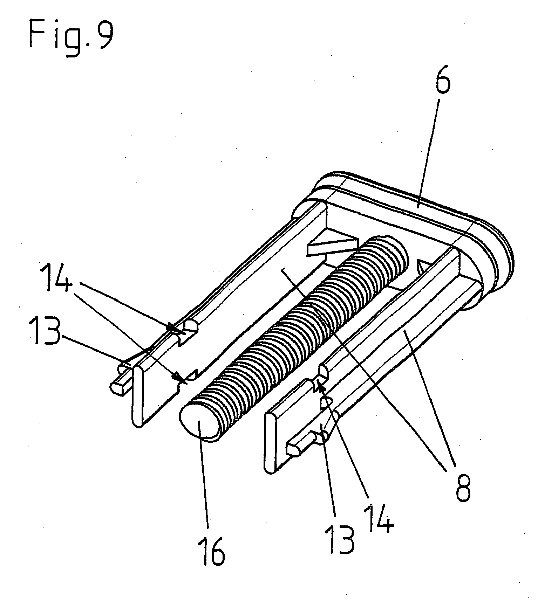

Fig. 9 zeigt eine Darstellung wie Fig. 4 einer bevorzugten Ausführungsvariante des Verdrängerkolbens 3 mit Tragkörper. Wie zu erkennen ist, weist diese Anordnung eine Gewindestange 16 auf, welche durch das Mutterngewinde eines Linearantriebs (nicht gezeigt) aufgenommen werden kann. Durch die Verbindung mit dem Linearantrieb lässt sich zudem auf einfache Weise eine Stopfenrückhalterung (Free Flow Prevention) realisieren.

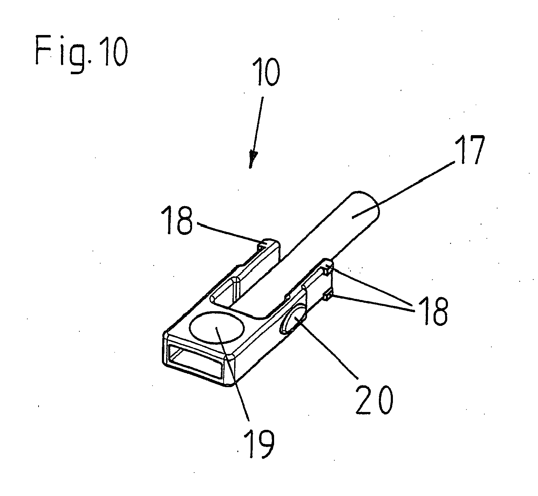

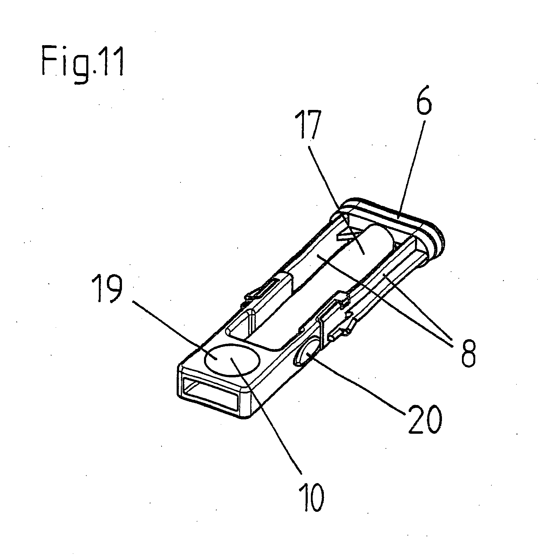

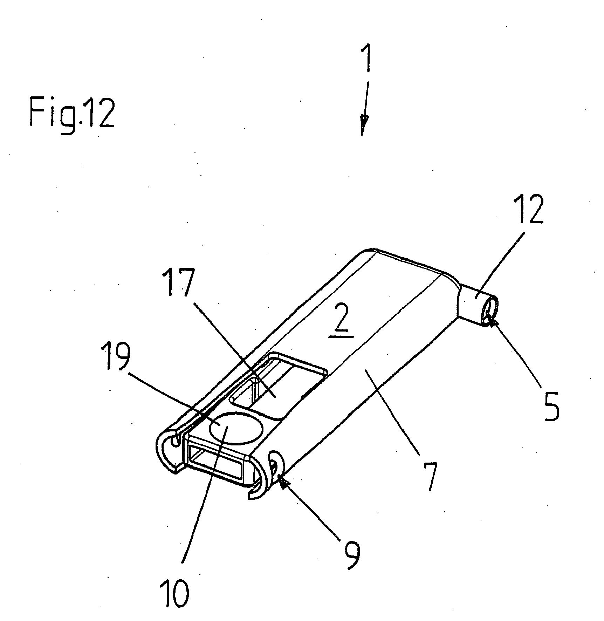

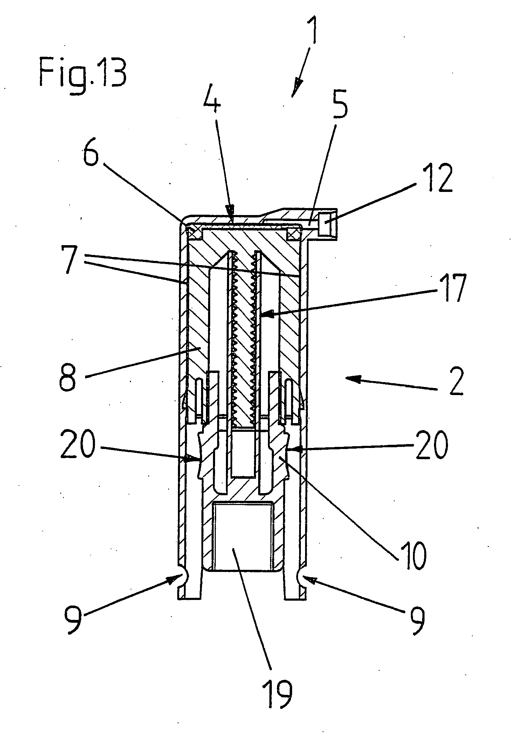

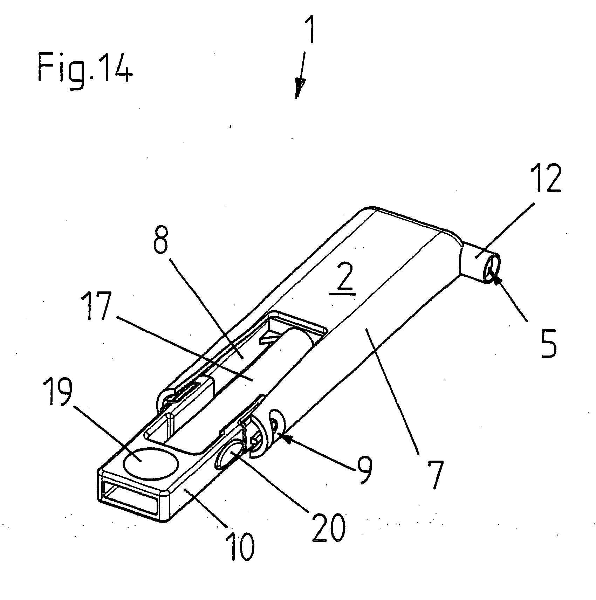

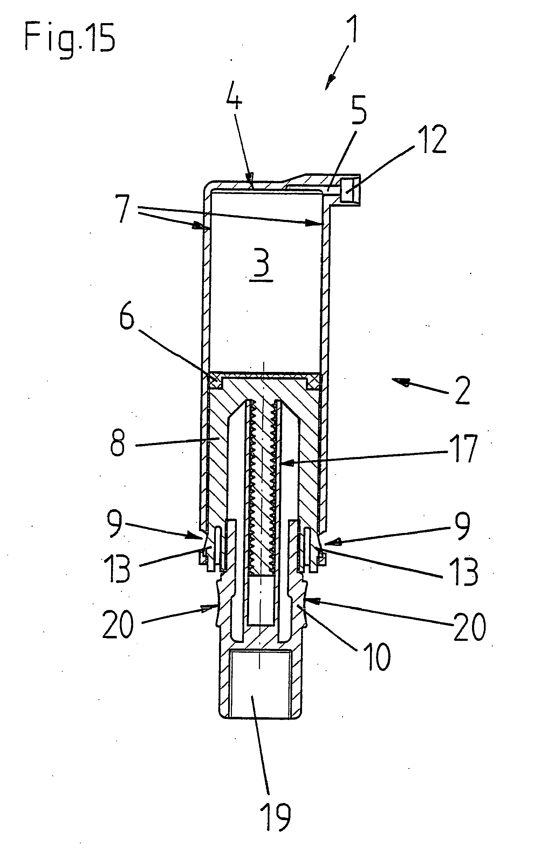

Fig. 10 zeigt eine perspektivische Draufsicht auf eine Aufziehhilfe 10 zur Befüllung des erfindungsgemässen Medikamentenreservoirs mit dem Verdrängerkolben gemäss Fig. 9. Die Aufziehhilfe 10 ist einstückig durch Spritzguss hergestellt und weist ein Schutzrohr 17 für die Gewindestange 16 des Verdrängerkolbens 6 auf. Wie zu erkennen ist, bildet die Aufziehhilfe 10 zwei Federarme, an deren freien Enden je zwei Rastnasen 18 zum Einrasten in die Ausnehmungen 14 der Kolbenstangen 8 gebildet sind. Zum Ankoppeln an das Medikamentenreservoir 1 wird die Aufziehhilfe 10 mit dem Schutzrohr 17 auf die Gewindestange 16 aufgeschoben, bis die Rastnasen 18 in die Ausnehmungen einrasten. Diese angekoppelte Situation ist in Fig. 11 perspektivisch ohne die Behältniswandungen, in Fig. 12 perspektivisch bei vollständigem Medikamentenreservoir 1 und in Fig. 13 bei vollständigem Medikamentenreservoir 1 im Schnitt dargestellt. Zum Befüllen wird sodann das Medikamentenreservoir 1 mit der einen Hand und die Aufziehhilfe 10 an deren Griffteil.19 mit der anderen Hand ergriffen und die Aufziehhilfe 10 mit dem daran angekoppelten Verdrängerkolben 6, 8 in einer Richtung entgegengesetzt der bestimmungsgemässen Förderbewegungsrichtung aus dem Behältnis 2 herausgezogen, bis die Federnasen 13 der Kolbenstangen 8 in den Ausnehmungen 9 der Begrenzungswandungen 7 des Behältnisses 2 einrasten und somit formschlüssig diese Bewegung begrenzen. Diese Situation ist in Fig. 14 perspektivisch und in Fig. 15 im Schnitt dargestellt. Durch Drücken der Entkopplungstasten 20 zwischen zwei Fingern können die Federarme, welche die Rastnasen 18 bilden, aufeinander zu bewegt werden, so dass die Rastnasen 18 ausser Eingriff mit den Ausnehmungen 14 gebracht werden und die Aufziehhilfe 10 entfernt werden kann.

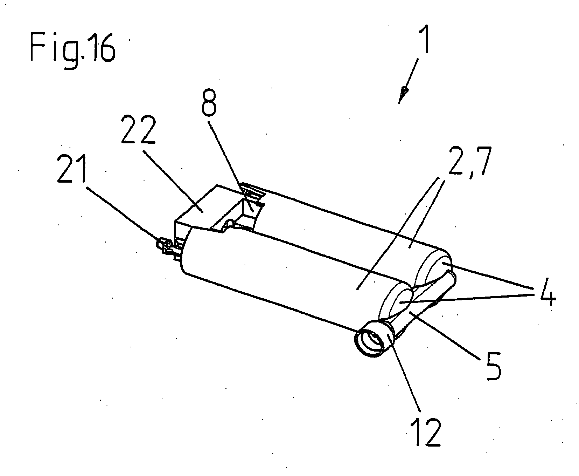

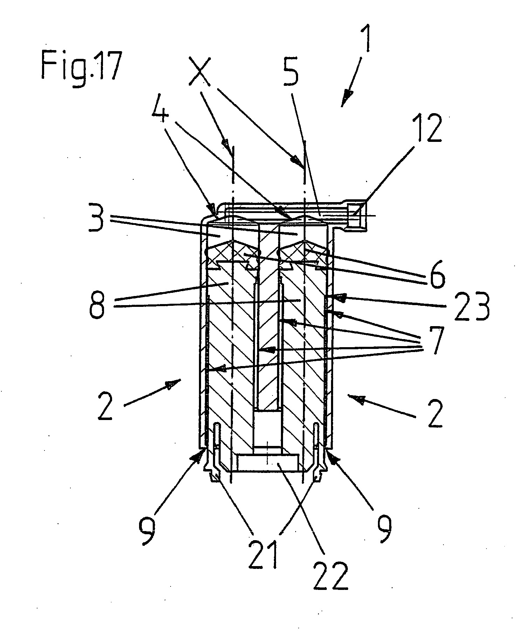

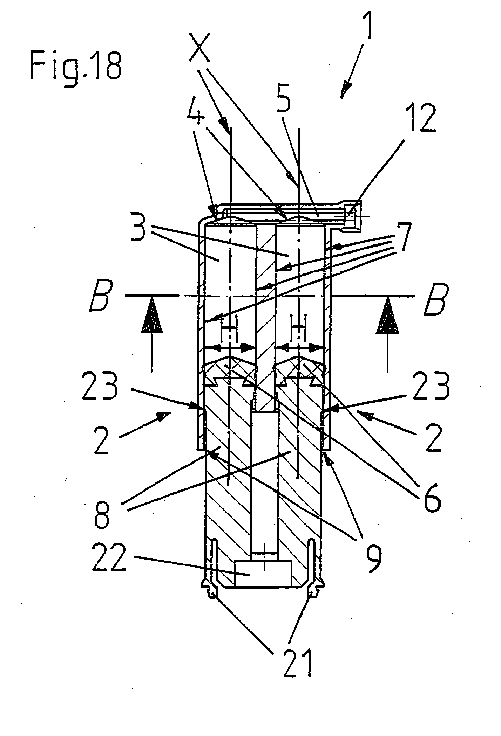

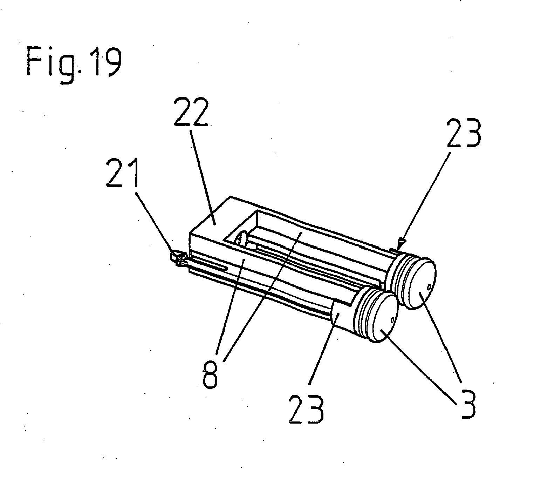

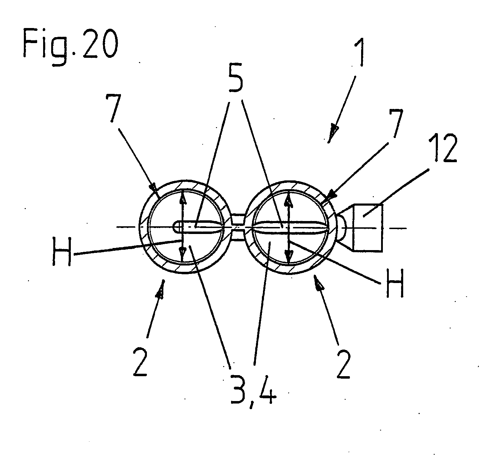

Die Figuren 16 bis 20 zeigen Darstellungen wie die Figuren 1 bis 5 einer zweiten bevorzugten Ausführungsform des erfindungsgemässen Medikamentenreservoirs, und zwar Fig. 16 eine perspektivische Draufsicht auf das Medikamentenreservoir, die Figuren 17 und 18 Längsschnitte durch das Medikamentenreservoir aus Fig. 16, einmal mit den Verdrängerkolben angeordnet in einer vorderen Verschiebeposition (Fig. 17) und einmal angeordnet in der hinteren Verschiebeposition (Fig. 18), Fig. 19 eine perspektivische Draufsicht auf die Verdrängerkolben des Medikamentenreservoirs sowie Fig. 20 einen Schnitt entlang der Linie B-B'in Fig. 18. Wie zu erkennen ist, umfasst das Medikamentenreservoir 1 hier zwei Behältnisse 2, innerhalb derer Begrenzungswandungen 7 jeweils ein Verdrängerkolben 6 längsverschiebbar angeordnet ist, zum Verdrängen von Flüssigkeit aus dem Behältnisinnenraum 3. Die Behältnisse 2 sind bezüglich ihrer Längsachsen X parallel angeordnet und weisen gleich grosse, kreisrunde Querschnitte und entsprechende kreisrunde Verdrängerkolben 6 auf. Die je Behältnis 2 kleinste Erstreckung der für die Verdrängung wirksamen Kolbenfläche entspricht hier dem jeweiligen Durchmesser des Behältnisquerschnitts bzw. dem Durchmesser des zugeordneten Verdrängerkolbens 6. Da die Durchmesser beider Behältnisse 2 bzw. Verdrängerkolben 6 identisch sind, entspricht die anspruchsgemässe grösste von den jeweils kleinsten Erstreckungen H der für die Verdrängung wirksamen Flächen der Verdrängerkolben 6 im vorliegenden Fall dem Durchmesser eines der beiden Verdrängerkolben 6 bzw. Behältnisse 2. Der Behältnisinnenraum 3 weist jeweils eine Stirnwand 4 auf, an welcher ein für beide Behältnisse 2 gemeinsamer Medikamentenaustritt 5 mit einer bestimmungsgemässen Austrittsrichtung, welche ebenfalls senkrecht zur Verschieberichtung des Verdrängerkolbens 6 bzw. zu den Längsachsen X der Behältnisse 2 verläuft, nach aussen führt. Der Medikamentenaustritt 5 mündet auch hier in einen Anschlussstutzen 12, an welchen eine Medikamentenförderleitung (nicht gezeigt) direkt anschliessbar ist. Die Verdrängerkolben 6 sind hier ebenfalls aus einem weichen Material gebildet und die jeweiligen, den Verdrängerkolben 6 zugeordneten Kolbenstangen 8 aus einem harten Material, wobei auch hier die Hart- 8 und Weichkomponenten 6 durch Mehrkomponentenspritzguss einstückig miteinander ausgebildet sind. Es ist jedoch auch hier denkbar, einen Tragkörper mit Kolbenteil aus dem harten Material herzustellen und am Umfang des Kolbenteils eine Dichtung, z.B. einen O-Ring, zur radialen Abdichtung gegenüber den Begrenzungswandungen 7 anzuordnen. Wie weiter zu erkennen ist, sind die beiden Kolbenstangen 8 der Verdrängerkolben 6 einstückig über eine Brücke 22 miteinander verbunden, derartig, dass beide Kolben zwangsläufig gleichzeitig und gleichartig miteinander bewegt werden müssen. Die Behältnisse 2 sind ebenfalls einstückig miteinander und mit dem den Medikamentenaustritt 5 bildenden Bauteil ausgebildet, was hier durch Spritzguss erfolgt ist.

Wie aus einer Zusammenschau der Figuren 18 und 19 erkennbar wird, weisen die Verdrängerkolben 6 bzw. deren Kolbenstangen 8 jeweils eine Anschlagschulter 23 auf, welche beim Bewegen des Verdrängerkolbens 6 entgegen der bestimmungsgemässen Förderbewegungsrichtung des Kolbens 6 an einem Anschlag 9 an den Begrenzungswandungen 7 des zugeordneten Behältnisses 2 anschlägt, und so den Verschiebeweg des jeweiligen Verdrängerkolbens 6 in einer Richtung entgegengesetzt der zur Medikamentenförderung vorgesehenen Verschieberichtung formschlüssig begrenzt. Gleichzeitig dienen die Anschlagschultern 23 der Führung der Anordnung bestehend aus den Verdrängerkolben 6, den Kolbenstangen 8 und der Brücke 22 an den Begrenzungswandungen 7 der Behältnisse 2, so dass ein Verkanten bzw. Abkippen der Verdrängerkolben 6 sicher verhindert wird. Wie weiter zu erkennen ist, weisen die beiden Kolbenstangen 8 im Bereich ihrer freien Enden jeweils eine Federnase 21 auf, welche sowohl zur Ankopplung einer Aufziehhilfe als auch zur Ankopplung einer Antriebseinheit für die Verdrängerkolben 6 dienen können.

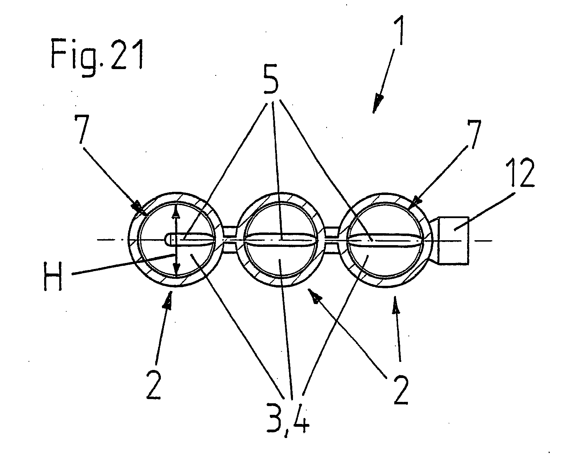

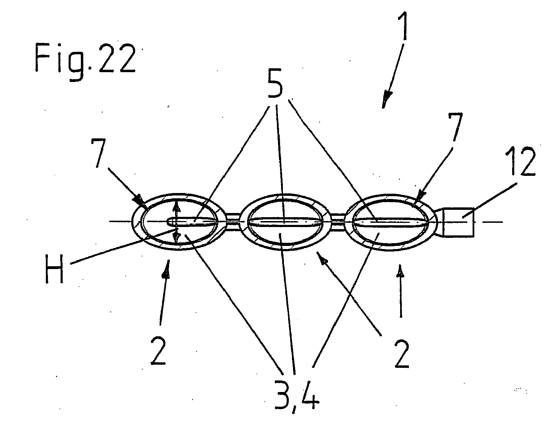

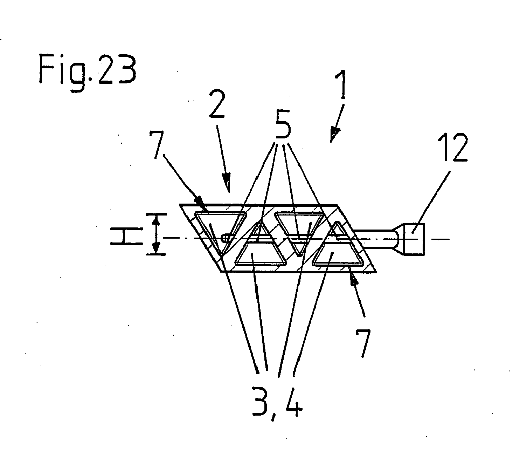

Die Figuren 21 bis 23 zeigen Darstellungen wie Fig. 20 durch bevorzugte Ausführungsvarianten des erfindungsgemässen Medikamentenreservoirs 1 aus Fig. 16, welche andere Konfigurationen und/oder Querschnittsformen aufweisen. Wie zu erkennen ist, weisen die Ausführungsformen gemäss den Figuren 21 und 22 jeweils drei identische Behältnisse 2 auf, einmal mit jeweils kreisrunder Querschnittsform (Fig. 21) und einmal mit jeweils ovaler Querschnittsform (Fig. 22). Die Fig. 23 zeigt eine Ausführungsform, bei welcher vier Behältnisse 2 mit identischen dreieckigen Querschnittsformen nebeneinander angeordnet sind, derart, dass abwechselnd einmal eine Spitze der Dreiecksform nach oben und einmal nach unten zeigt. Bei allen Ausführungsformen sind die Behältnisse 2 einstückig miteinander und mit dem den gemeinsamen Medikamentenaustritt 5 bildenden Bauteil ausgebildet. Bei diesen Ausführungsformen ergeben sich die anspruchsgemässen grössten von den jeweils kleinsten Erstreckungen H der für die Verdrängung wirksamen Flächen der Verdrängerkolben einmal als der Durchmesser eines der Behältnisses 2 bzw. des zugehörigen Verdrängerkolbens (Fig. 21), einmal als die Höhe des Querschnitts eines der Behältnisse 2 entlang der kürzesten Symmetrieachse der ovalen Querschnittsform (Fig. 22) und einmal als die Höhe des dreieckigen Querschnitts eines der Behältnisse 2 (Fig. 23). Die Ausführungsform gemäss Fig. 7 weist eine gestreckt hexagonale Querschnittsform auf, wobei hier die kleinste Ersteckung H der für die Verdrängung wirksamen Fläche des Verdrängerkolbens der Abstand zwischen den längsten parallelen Begrenzungswandungen 7 ist. Die übrige Ausgestaltung dieser Medikamentenreservoirs ist, abgesehen von der anderen Konfiguration bzw. Querschnittsform der Behältnisse 2, identisch zu der in den Figuren 16 bis 20 gezeigten.

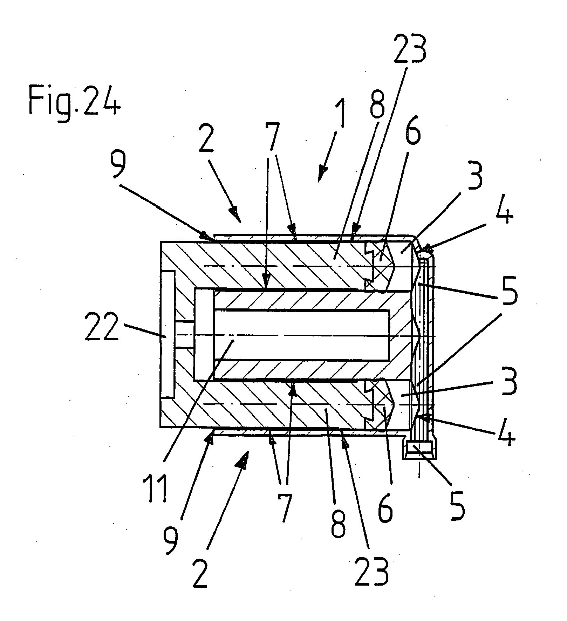

Fig. 24 zeigt einen Längsschnitt durch ein drittes erfindungsgemässes Medikamentenreservoir 1, welches sich von dem in Fig. 17 gezeigten lediglich dadurch unterscheidet, dass zwischen den Behältnissen 2 ein Raum 11 zur Aufnahme einer Antriebseinheit (nicht gezeigt) einer Insulinpumpe vorhanden ist und die beiden Kolbenstangen 8 im Bereich ihrer freien Enden keine Federnasen zur Ankopplung einer Aufziehhilfe bzw. einer Antriebseinheit aufweisen. Die übrige Ausgestaltung dieser Medikamentenreservoirs 1 ist identisch zu der in den Figuren 16 bis 20 gezeigten.

## Patentansprüche

1. Medikamentenreservoir (1), umfassend ein oder mehrere Behältnisse (2) zur Aufnahme eines flüssigen Medikaments oder enthaltend ein flüssiges Medikament, welche Behältnisse (2) jeweils einen Behältnisinnenraum (3), einen an einer Stirnwand (4) des Behältnisinnenraumes (3) nach aussen führenden Medikamentenaustritt (5) sowie einen den Behältnisinnenraum (3) nach aussen separierenden Verdrängerkolben (6), der in radialer Richtung über seinen gesamten Aussenumfang gesehen fluiddicht an diesen umschliessende Begrenzungswandungen (7) des Behältnisses (2) angrenzt, und der innerhalb des Behältnisses (2) unter Beibehaltung seiner fluiddichten Angrenzung an die Begrenzungswandungen (7) längsverschiebbar ist, zur Ermöglichung einer Förderung von flüssigem Medikament aus dem Behältnisinnenraum (3) in den Medikamentenaustritt (5) durch Verdrängung mittels des Verdrängerkolbens (6), wobei der Verdrängerkolben (6) in einer Verschiebeendposition, welche er am Ende der Medikamtenförderung bei maximal entleertem Medikamentenreservoir einnimmt, an die Stirnwand (4) des Behältnisses (2) angrenzt, an welcher der Medikamentenaustritt (5) nach aussen führt, **dadurch gekennzeichnet, dass** die bestimmungsgemässe Austrittsrichtung (A) aus dem Medikamentenaustritt (5) quer, insbesondere senkrecht zu einer Verschieberichtung des oder der Verdrängerkolben (6) verläuft.

2. Medikamentenreservoir (1) nach Anspruch 1, **dadurch gekennzeichnet, dass** die für die Verdrängung wirksame Fläche des oder der Verdrängerkolben (6) jeweils die vom Aussenumfang des Verdrängerkolbens umschlossene Fläche ist.

3. Medikamentenreservoir (1) nach einem der vorangehenden Ansprüche, **dadurch gekennzeichnet, dass** eine Medikamentenförderleitung direkt an den Medikamentenaustritt (5) des Medikamentenreservoirs (1) anschliessbar oder angeschlossen ist.

4. Medikamentenreservoir nach einem der vorangehenden Ansprüche, **dadurch gekennzeichnet, dass** die kleinste Erstreckung (H) der für die Verdrängung wirksamen Fläche des Verdrängerkolbens (6) oder, bei mehreren Behältnissen (2), die grösste von den jeweils kleinsten Erstreckungen (H) der für die Verdrängung wirksamen Flächen der Verdrängerkolben (6) kleiner ist als der Durchmesser eines Zylinders, welcher bei einer Höhe entsprechend dem für die Medikamentenförderung vorgesehenen Verschiebeweg (V) des Verdrängerkolbens (6) oder, bei mehreren Behältnissen (2), entsprechend dem längsten der für die Medikamentenförderung vorgesehenen Verschiebewege (V) der Verdrängerkolben (6) ein Volumen entsprechend dem vorgesehenen Fördervolumen des Medikamentenreservoirs (1) aufweist.

5. Medikamentenreservoir (1) nach einem der vorangehenden Ansprüche, **dadurch gekennzeichnet, dass** das Medikamentenreservoir (1) genau ein Behältnis (2) aufweist.

6. Medikamentenreservoir (1) nach einem der Ansprüche 1 bis 4, **dadurch gekennzeichnet, dass** das Medikamentenreservoir (1) mehrere, insbesondere identische Behältnisse (2) umfasst, insbesondere zwei oder drei insbesondere identische Behältnisse (2).

7. Medikamentenreservoir (1) nach einem der vorangehenden Ansprüche, **dadurch gekennzeichnet, dass** zumindest ein Behältnis (2), insbesondere alle Behältnisse (2) des Medikamentenreservoirs (1) jeweils einen Verdrängerkolben (6) aufweisen, dessen für die Verdrängung wirksame Fläche kreisrund ist.

8. Medikamentenreservoir (1) nach einem der vorangehenden Ansprüche, **dadurch gekennzeichnet, dass** zumindest ein Behältnis (2), insbesondere alle Behältnisse (2) des Medikamentenreservoirs (1) jeweils einen Verdrängerkolben (6) aufweisen, dessen für die Verdrängung wirksame Fläche eine nicht-kreisrunde Fläche ist, insbesondere eine ovale, knochenförmige, langlochförmige, rechteckige, dreieckige oder hexagonale Fläche ist.

9. Medikamentenreservoir (1) nach einem der vorangehenden Ansprüche mit mindestens zwei Behältnissen (2), **dadurch gekennzeichnet, dass** die Verdrängerkolben (6) der Behältnisse (2) mechanisch miteinander gekoppelt sind, insbesondere derart, dass deren Verschiebung zwangsläufig gleichzeitig erfolgen muss.

10. Medikamentenreservoir (1) nach einem der vorangehenden Ansprüche mit mindestens zwei Behältnissen (2), **dadurch gekennzeichnet, dass** die Behältnisse (2) mit ihren Längsachsen (X) parallel zueinander angeordnet sind, insbesondere derart, dass die Längsachsen sämtlicher Behältnisse (2) in einer Ebene angeordnet sind.

11. Medikamentenreservoir (1) nach einem der vorangehenden Ansprüche mit mindestens zwei Behältnissen (2), **dadurch gekennzeichnet, dass** die Behältnisse (2) fluidisch miteinander verbunden sind.

12. Medikamentenreservoir (1) nach einem der vorangehenden Ansprüche mit mindestens zwei Behältnissen (2), **dadurch gekennzeichnet, dass** die Behältnisse (2) einstückig miteinander ausgebildet sind, und insbesondere, dass diese einen gemeinsamen, einstückig mit diesen ausgebildeten Medikamentenaustritt (5) aufweisen.

13. Medikamentenreservoir (1) nach einem der vorangehenden Ansprüche mit mindestens zwei Behältnissen (2), **dadurch gekennzeichnet, dass** die Behältnisse (2) als separate Bauteile ausgebildet sind, und insbesondere, dass diese über ein weiteres Bauteil fluidisch miteinander gekoppelt sind, insbesondere derart, dass deren Medikamentenaustritte (5) miteinander kommunizieren.

14. Medikamentenreservoir (1) nach einem der vorangehenden Ansprüche mit mindestens zwei Behältnissen (2), **dadurch gekennzeichnet, dass** zwischen zwei benachbarten Behältnissen (2) ein Zwischenraum vorhanden ist.

15. Medikamentenreservoir (1) nach einem der vorangehenden Ansprüche, **dadurch gekennzeichnet, dass** jeder Verdrängerkolben (6) des Medikamentenreservoirs (1) insbesondere durch einstückige Ausbildung mit einer zugeordneten Kolbenstange (8) gekoppelt ist.

16. Medikamentenreservoir (1) nach Anspruch 15, **dadurch gekennzeichnet, dass** die Kolbenstange (8) im Medikamentenreservoir (1) radial geführt ist.

17. Medikamentenreservoir (1) nach einem der Ansprüche 15 bis 16, **dadurch gekennzeichnet, dass** die Kolbenstange (8) aus einem härteren Material gebildet ist als der Verdrängerkolben (6), und insbesondere, dass Kolbenstange (8) und Verdrängerkolben (6) durch Mehrkomponentenspritzguss einstückig miteinander ausgebildet sind.

18. Medikamentenreservoir nach einem der Ansprüche 15 bis 17 mit mindestens zwei Behältnissen (2), **dadurch gekennzeichnet, dass** die Kolbenstangen (8) der Verdrängerkolben (6) insbesondere durch einstückige Ausbildung derartig miteinander verbunden sind, dass ein Verschieben der Verdrängerkolben (6) zwangsläufig gleichzeitig um den gleichen Betrag erfolgen muss.

19. Medikamentenreservoir (1) nach einem der vorangehenden Ansprüche, **dadurch gekennzeichnet, dass** Anschlagmittel (9) vorhanden sind, welche den Verschiebeweg (V) des oder der Verdrängerkolben (6) in einer Richtung entgegen der zur Medikamentenförderung vorgesehenen Verschieberichtung formschlüssig begrenzen.

20. Medikamentenreservoir (1) nach einem der vorangehenden Ansprüche, **dadurch gekennzeichnet, dass** dieses mit Insulin befüllt ist, insbesondere mit einer Insulinmenge entsprechend mindestens 150 IU, insbesondere entsprechend mindestens 300 IU.

21. Vorrichtung zum automatisierten Abgeben eines flüssigen Medikaments, umfassend ein Medikamentenreservoir (1) nach einem der vorangehenden Ansprüche zur Bereitstellung eines flüssigen Medikaments sowie eine mit einer insbesondere elektronischen Steuerung versehene Antriebseinheit, mittels welcher der oder die Verdrängerkolben (6) des Medikamentenreservoirs (1) im jeweiligen sie aufnehmenden Behältnis (2) verschiebbar sind zur Bewirkung einer Förderung von flüssigem Medikament aus dem Behältnis (2).

22. Vorrichtung nach Anspruch 21, **dadurch gekennzeichnet, dass** die Anschlussrichtung einer Medikamentenförderleitung an die Vorrichtung quer, insbesondere senkrecht zur Verschieberichtung des oder der Verdrängerkolben (6) verläuft, und insbesondere, dass die Medikamentenförderleitung direkt an den Medikamentenaustritt (5) des Medikamentenreservoirs (1) anschliessbar oder angeschlossen ist.

23. Vorrichtung nach einem der Ansprüche 21 bis 22 mit einem Medikamentenreservoir (1) mit mindestens zwei Behältnissen (2), **dadurch gekennzeichnet, dass** die Verdrängerkolben (6) der Behältnisse (2) derartig mechanisch miteinander gekoppelt sind, dass deren Verschiebung zwangsläufig gleichzeitig erfolgen muss, insbesondere um einen identischen Betrag.

24. Vorrichtung nach einem der Ansprüche 21 bis 23 mit einem Medikamentenreservoir (1) mit mindestens zwei Behältnissen (2), **dadurch gekennzeichnet, dass** zwischen zwei benachbarten Behältnissen (2) des Medikamentenreservoirs (1) ein Zwischenraum (10) vorhanden ist, in welchem zumindest ein Teil der Antriebseinheit angeordnet ist, insbesondere ein Antriebsmotor und/oder ein Getriebe der Antriebseinheit.

25. Vorrichtung nach einem der Ansprüche 21 bis 24, **dadurch gekennzeichnet, dass** die Vorrichtung eine am Körper eines Patienten tragbare Insulinpumpe ist.

26. Set umfassend ein Medikamentenreservoir (1) nach einem der Ansprüche 1 bis 20 und eine Aufziehhilfe (11) zum Ankoppeln an das Medikamentenreservoir (1) zum Zurückziehen des oder der Verdrängerkolben (6) zwecks Befüllung des Medikamentenreservoirs (1).
